# FASCICULE DE BREVET EUROPEEN

(11) **EP 3 055 006 B1**
(45) Date de publication et mention de la délivrance du brevet: **24.12.2025**
(21) Numéro de dépôt: 14799188.9
(22) Date de dépôt: 07.10.2014
(51) Int. Cl.: A61M 5/315

(54) **BOUCHON-PISTON GLISSANT ET DISPOSITIF DE SERINGUE COMPORTANT UN TEL BOUCHON-PISTON**
GLEITENDE KOLBENSTANGE SPRITZENVORRICHTUNG MIT EINER SOLCHEN KOLBENSTANGE
SLIDING PISTON ROD AND SYRINGE DEVICE COMPRISING SUCH PISTON ROD

(30) Priorité: 09.10.2013 FR 1359773
(43) Date de publication de la demande: 17.08.2016
(73) Titulaire: Aptar Stelmi SAS, 93420 Villepinte (FR)
(72) Inventeur: FOURNIER, Ghislain, F-17000 La Rochelle (FR); SWAL, Mickaël, F-77124 Chauconin Neufmontiers (FR)
(74) Mandataire: CAPRI
(86) Numéro de dépôt international: PCT/FR2014/052540
(87) Numéro de publication internationale: WO 2015/052429

(56) Documents cités:
- EP-A1- 1 180 377
- EP-A1- 1 674 121
- WO-A1-02/092312
- WO-A1-2006/021380
- WO-A1-2012/134430
- DE-A1- 10 006 560
- DE-U1- 202008 011 890
- US-A1- 2011 204 097

## Description

La présente invention concerne un bouchon-piston et un dispositif de seringue comportant un tel bouchon-piston.

Le bouchon-piston pour dispositif de seringue est bien connu dans l'état de la technique. Avant actionnement de la seringue, il remplit la fonction de bouchon en isolant le produit fluide contenu dans le corps de seringue, et lors de l'actionnement il se transforme en piston en poussant le produit fluide hors du corps de seringue, généralement à travers une aiguille. Généralement, le dispositif de seringue comporte un organe d'actionnement, tel qu'une tige de piston, qui coopère avec ledit bouchon-piston pour le déplacer dans le corps de seringue lors de l'actionnement. Ce bouchon-piston doit donc assurer l'étanchéité et est donc généralement réalisé en caoutchouc ou autre élastomère similaire. Un inconvénient avec ce type de matériau concerne les risques d'interactions entre le matériau du bouchon-piston et le produit fluide à distribuer, notamment pendant la phase de stockage, ces interactions pouvant altérer ledit produit fluide. Pour limiter ces risques d'interactions, il a été proposé de revêtir la surface frontale du bouchon-piston, c'est-à-dire la surface en contact avec le produit fluide pendant le stockage et pendant la distribution du produit, avec un revêtement approprié. Il a ainsi notamment été proposé de disposer un film mince en ETFE (éthylène tétrafluoroéthylène) sur la surface frontale du bouchon-piston. Cette mise en œuvre permet de limiter les risques d'interactions entre le matériau du bouchon-piston et le produit fluide, mais la présence de ce revêtement sur la surface frontale rigidifie celle-ci, et rend donc l'assemblage et le déplacement du bouchon-piston dans le corps de seringue plus difficile. Plus précisément, la présence de ce revêtement frontal diminue la capacité de déformation de la surface frontale du bouchon-piston et rend donc son insertion et son glissement dans le corps de seringue plus difficile. Il se pose donc le problème de trouver le bon compromis entre une étanchéité suffisante pour le bouchon-piston une fois inséré dans le corps de seringue et un processus d'assemblage et d'actionnement qui ne soit pas trop compliqué ou difficile.

Par ailleurs, la coopération entre le bouchon-piston et l'organe d'actionnement, en général une tige de piston, peut se faire généralement par l'intermédiaire d'un filetage prévu sur la surface interne du bouchon-piston et qui coopère avec un filet correspondant aménagé sur l'extrémité avant de la tige de piston. Cette mise en œuvre complique également la fabrication du bouchon piston, notamment la phase de démoulage, en raison des contre-dépouilles importantes du moule de fabrication du bouchon piston nécessaires pour mouler le filetage.

Les documents WO2006021380, EP1180377, EP1674121, DE10006560 et WO02092312 décrivent des dispositifs de l'état de la technique.

La présente invention a pour but de fournir un bouchon-piston qui ne reproduise pas les inconvénients susmentionnés.

En particulier, la présente invention a pour but de fournir un bouchon-piston qui garantisse une parfaite étanchéité lors du stockage et lors de l'actionnement, tout en permettant un procédé d'assemblage et un actionnement simplifié et fiable.

La présente invention a également pour but de fournir un bouchon-piston qui soit plus simple et donc moins coûteux à fabriquer et à assembler, notamment dans le processus de moulage dudit bouchon-piston et lors de l'insertion du piston-bouchon dans un corps de seringue.

La présente invention a donc pour objet un bouchon-piston, notamment en élastomère, comportant un corps cylindrique ayant un côté axial avant fermé par une paroi frontale, ledit corps cylindrique comportant une surface externe pourvue d'au moins un profil d'étanchéité, ladite paroi frontale comportant une surface externe axiale frontale pourvue d'un revêtement, avantageusement un film en éthylène tétrafluoroéthylène (ETFE), ladite paroi frontale comportant sur son bord radialement externe un profil d'étanchéité, ladite surface externe axiale frontale de ladite paroi frontale comportant un profil de déformation disposé radialement à l'intérieur dudit profil d'étanchéité de ladite paroi frontale, ledit profil de déformation étant adapté à se déformer radialement pour faciliter l'insertion et/ou le glissement dudit bouchon-piston dans un corps de seringue.

Avantageusement, ledit corps cylindrique creux comporte une pluralité de profils d'étanchéité, avantageusement deux.

Avantageusement, ledit au moins un profil d'étanchéité dudit corps cylindrique creux est un bourrelet radialement saillant.

Avantageusement, ledit profil de déformation est réalisé sous la forme d'une rainure périphérique ménagée dans ladite surface externe axiale frontale de ladite paroi frontale, ladite rainure étant avantageusement en forme de V ou de U.

Avantageusement, ledit profil d'étanchéité de ladite paroi frontale est formé entre ledit profil de déformation et un évidement périphérique radialement externe axialement décalé vers l'arrière par rapport à ladite surface externe axiale frontale, ledit évidement périphérique radialement externe étant également déformé lors de l'insertion et/ou l'actionnement dudit bouchon-piston dans un corps de seringue.

Avantageusement, ledit corps cylindrique est creux et définit un volume interne, ledit corps cylindrique creux ayant un côté axial arrière ouvert axialement opposé audit côté axial avant fermé par ladite paroi frontale.

Avantageusement, ledit volume interne est adapté à coopérer avec un organe d'actionnement d'un dispositif de seringue.

Avantageusement, ledit corps cylindrique creux comporte sur sa surface interne un filetage adapté à se visser sur un filet dudit organe d'actionnement.

Avantageusement, ledit filetage est discontinu, une pluralité de rainures axiales, avantageusement quatre, réalisées dans la surface interne dudit corps cylindrique creux, étant prévues pour interrompre ledit filetage.

Avantageusement, ladite surface externe axiale frontale est conique et définit une pointe axiale centrale.

En variante, ladite surface externe axiale frontale est sensiblement plane et perpendiculaire à un axe central dudit piston-bouchon.

Avantageusement, ledit profil de déformation est de forme arrondie, de sorte que ladite surface externe axiale frontale de ladite paroi frontale est dépourvue d'angles vifs.

La présente invention a aussi pour objet un dispositif de seringue, comportant un corps de seringue cylindrique contenant un produit fluide, ledit corps de seringue recevant de manière coulissante un bouchon-piston tel que décrit ci-dessus.

Avantageusement, ledit au moins un profil d'étanchéité dudit corps cylindrique creux et ledit profil d'étanchéité de ladite paroi frontale coopèrent de manière étanche avec la surface cylindrique interne dudit corps de seringue, ladite surface externe axiale frontale étant en contact avec ledit produit fluide.

Avantageusement, ledit profil d'étanchéité est au moins partiellement pourvu dudit revêtement, définissant ainsi une zone d'étanchéité cylindrique entre la surface cylindrique interne dudit corps de seringue et ledit profil d'étanchéité revêtu.

La présente invention a aussi pour objet une machine de fabrication d'un piston-bouchon tel que décrit ci-dessus, comportant une première partie de moule et une seconde partie de moule, ladite première partie de moule définissant la forme dudit corps cylindrique creux et ladite seconde partie de moule définissant la forme de ladite paroi frontale.

Avantageusement, ladite première partie de moule comporte un noyau comportant un corps prolongé par un embout axial définissant ledit volume interne, ledit embout axial étant pourvu d'un filet externe séparé par plusieurs, avantageusement quatre, nervures axiales, définissant un filetage et des rainures axiales dudit bouchon-piston.

Ces caractéristiques et avantages et d'autres de la présente invention apparaîtront plus clairement au cours de la description détaillée suivante, faite en référence aux dessins joints, donnés à titre d'exemples non-limitatifs, et sur lesquels :
la figure 1 est une vue schématique en section transversale d'un bouchon-piston selon un premier mode de réalisation avantageux de la présente invention,
la figure 2 est une vue similaire à celle de la figure 1, illustrant le bouchon-piston après son insertion dans un corps de seringue,
la figure 3 illustre schématiquement les différentes étapes du procédé d'insertion du bouchon-piston de la figure 1 dans un corps de seringue,
la figure 4 est une vue similaire à celle de la figure 1, illustrant un second mode de réalisation avantageux de la présente invention,
la figure 5 est une vue schématique en perspective d'un noyau de moule selon une première variante de réalisation,
la figure 6 est une vue similaire à celle de la figure 5, montrant un noyau de moule adapté à la fabrication d'un bouchon-piston selon une seconde variante de réalisation,
la figure 7 est une vue schématique en section transversale des deux parties de moule utilisées pour fabriquer le bouchon-piston de la figure 1,
la figure 8 est une vue schématique en perspective de dessus d'une partie de moule de la figure 7,
les figures 9 et 10 sont des graphiques comparant les forces engendrées par des pistons coulissant dans des tubes, respectivement sans revêtement frontal ETFE et avec revêtement frontal ETFE,
les figures 11 et 12 sont des vues similaires à celles des figures 1 et 2, illustrant une variante de réalisation avec une surface frontale conique, et
les figures 13 et 14 sont des vues similaires à celles de la figure 4, illustrant deux variantes de réalisation avec une surface frontale plane.

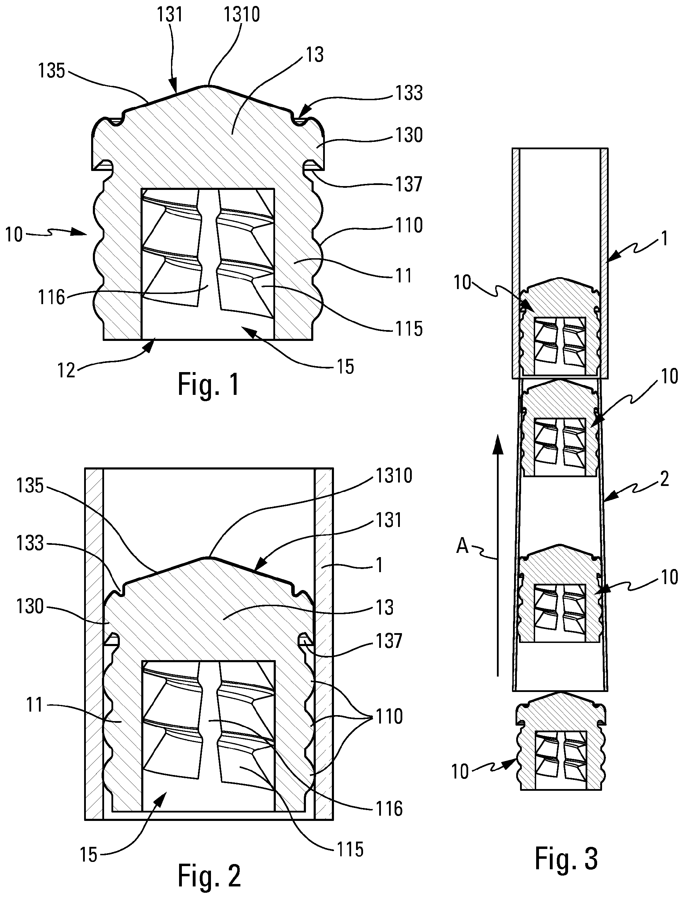

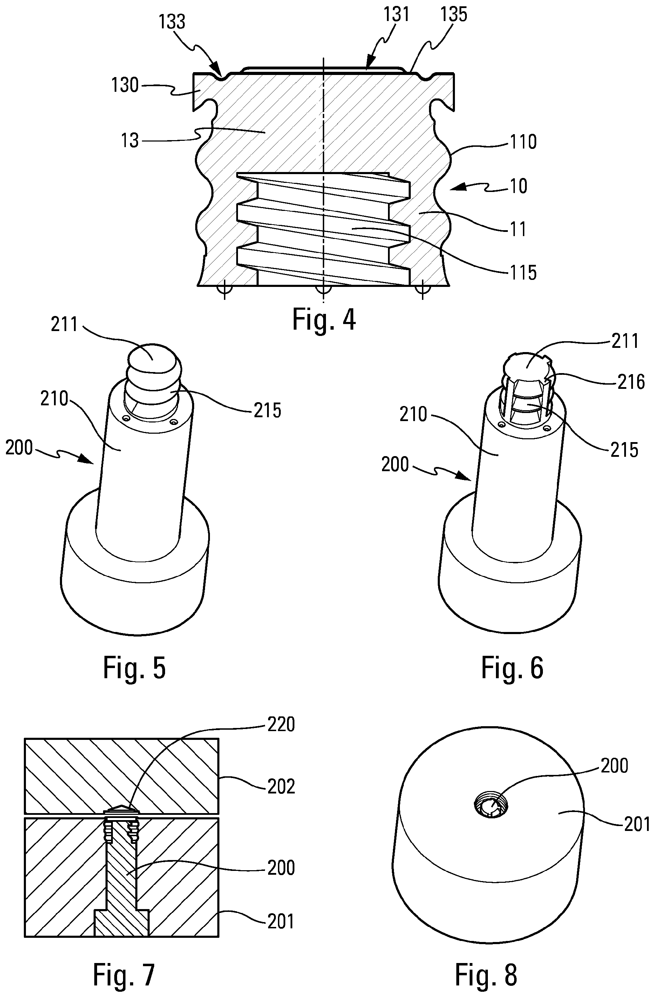

Les figures 1 à 3 illustrent un premier mode de réalisation de l'invention. Dans ce premier mode de réalisation, le bouchon-piston 10, réalisé en élastomère, en caoutchouc ou en tout autre matériau approprié, comporte un corps cylindrique creux 11. Ce corps cylindrique creux comporte un côté axial arrière ouvert 12 et un côté axial avant qui est fermé par une paroi frontale 13. Ainsi, cette mise en œuvre définit un bouchon-piston 10 de forme généralement cylindrique avec un volume interne 15 borgne, c'est-à-dire ouvert d'un côté et fermé de l'autre côté. La paroi frontale 13 définit une surface externe axiale frontale 131 qui est la surface qui sera en contact du produit fluide lorsque le bouchon-piston 10 sera assemblé dans un corps de seringue 1 destiné à contenir un tel produit fluide. Cette surface externe axiale frontale 131 est pourvue d'un revêtement 135, avantageusement un film mince réalisé en ETFE (éthylène tétrafluoroéthylène), afin de limiter au maximum les interactions entre ledit produit fluide et le matériau dudit bouchon-piston. D'autres types de revêtements appropriés sont aussi envisageables, aussi bien en ce qui concerne le mode d'application du revêtement que le matériau constituant ledit revêtement. Cette surface externe axiale frontale 131 peut être conique en définissant une pointe axiale 1310, comme visible sur les modes de réalisation des figures 1 à 3, 11 et 12, mais elle peut également être plane et sensiblement perpendiculaire à l'axe central du bouchon-piston, comme représenté sur les figures 4, 13 et 14.

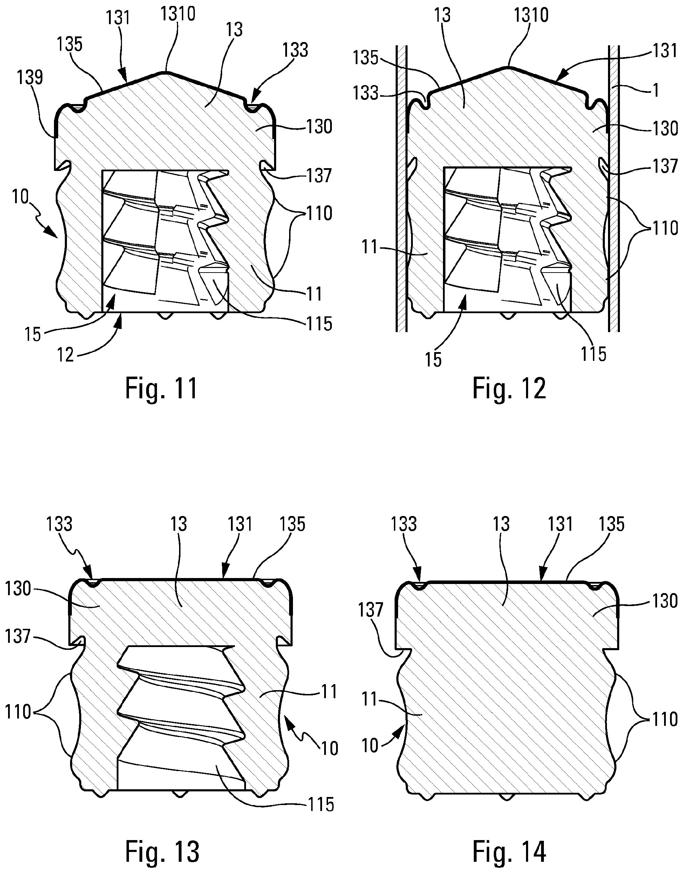

La surface externe du corps cylindrique creux 11 comporte au moins un, de préférence plusieurs profils d'étanchéité 110, qui sont généralement réalisés sous la forme de bourrelets radialement saillants. De préférence, comme représenté sur les figures 11 et 12, la surface externe du corps cylindrique creux 11 comporte deux profils d'étanchéité 110 réalisés sous la forme de bourrelets radialement saillants. La paroi frontale 13 comporte également un profil d'étanchéité 130 réalisé sur son bord radialement externe.

Selon la présente invention, ladite surface externe axiale frontale 131 de ladite paroi frontale 13 comporte un profil de déformation 133 qui est adapté à se déformer pour faciliter l'insertion du bouchon-piston 10 dans un corps de seringue 1. Ce profil de déformation est disposé radialement à l'intérieur dudit profil d'étanchéité 130, c'est-à-dire qu'il n'est pas disposé au niveau du bord radialement externe de ladite paroi frontale 13. Avantageusement, ce profil de déformation 133 est réalisé sous la forme d'une rainure périphérique ménagée dans ladite surface externe axiale frontale 131. Dans l'exemple représenté sur la figure 1, cette rainure, avant déformation, est sensiblement en forme de U, mais elle pourrait évidemment également avoir une autre forme, par exemple une forme en V, en W ou toute autre forme appropriée permettant une déformation radiale de la paroi frontale 13 lors de l'insertion du bouchon-piston.

De manière avantageuse, ledit profil de déformation 133 est de forme arrondie, c'est-à-dire qu'il ne définit pas d'angle vif dans ladite surface externe axiale frontale 131 de ladite paroi frontale 13. Or, de tels angles vifs peuvent générer des zones de faiblesse du revêtement 135, non seulement pendant le moulage du bouchon-piston où ils peuvent être générateur d'arrachée, mais aussi en utilisation, où le revêtement 135 pourrait se fissurer ou se déchirer au niveau desdits angles vifs, par exemple lors de la compression du bouchon-piston pendant sa mise en place dans la seringue, avec par conséquent des risques de discontinuité dudit revêtement.

Avantageusement, ledit revêtement 135 s'étend non seulement sur ladite surface externe axiale frontale 131, mais également au moins partiellement sur la surface cylindrique externe dudit profil d'étanchéité 130, comme visible plus clairement sur les figures 11 et 12. Avantageusement, ce revêtement 135 s'étend jusqu'à un épaulement 139 dudit profil d'étanchéité 130. Ceci permet de garantir la présence d'une première zone d'étanchéité cylindrique entre l'intérieur du corps de seringue 1 et la partie revêtue dudit profil d'étanchéité 130 du bouchon-piston 10. Ceci améliore en particulier l'étanchéité lors de fortes pressions exercées dans la seringue. En effet, une zone de contact cylindrique entre le corps de seringue 1 et la zone revêtue du bouchon- piston 10 garantit l'absence de micro-pliures du revêtement 135 et permet ainsi d'avoir une zone d'étanchéité de contact constante entre le revêtement 135 du bouchon-piston et le corps de seringue 1.

La figure 2 montre le bouchon-piston 10 après son insertion dans un corps de seringue 1. On constate que le profil de déformation 133 s'est déformé radialement, ce qui a permis au profil d'étanchéité 130 de la paroi frontale 13 de pénétrer plus facilement à l'intérieur du corps de seringue 1.

La figure 3 illustre les différentes étapes du processus d'insertion. En bas de la figure 3 on voit le bouchon-piston 10 disposé à l'extérieur d'un cône d'insertion 2, avec un profil de déformation non déformé. Le bouchon-piston 10 est alors inséré progressivement à l'intérieur dudit cône d'insertion 2 dans le sens de la flèche A, et au fur et à mesure qu'il avance à l'intérieur dudit cône d'insertion, sa paroi frontale 13 va se déformer radialement jusqu'à atteindre une déformation maximale, avec laquelle il peut facilement être inséré à l'intérieur du corps de seringue 1, représenté en haut de la figure 3. Après insertion dudit bouchon-piston 10 dans le corps de seringue 1, les différents profils d'étanchéité, c'est-à-dire le profil d'étanchéité 130 de la paroi frontale 13 et les profils d'étanchéité 110 du corps cylindrique creux 11 peuvent légèrement se détendre tout en restant suffisamment contraints contre la paroi cylindrique du corps de seringue 1 afin d'assurer une parfaite étanchéité.

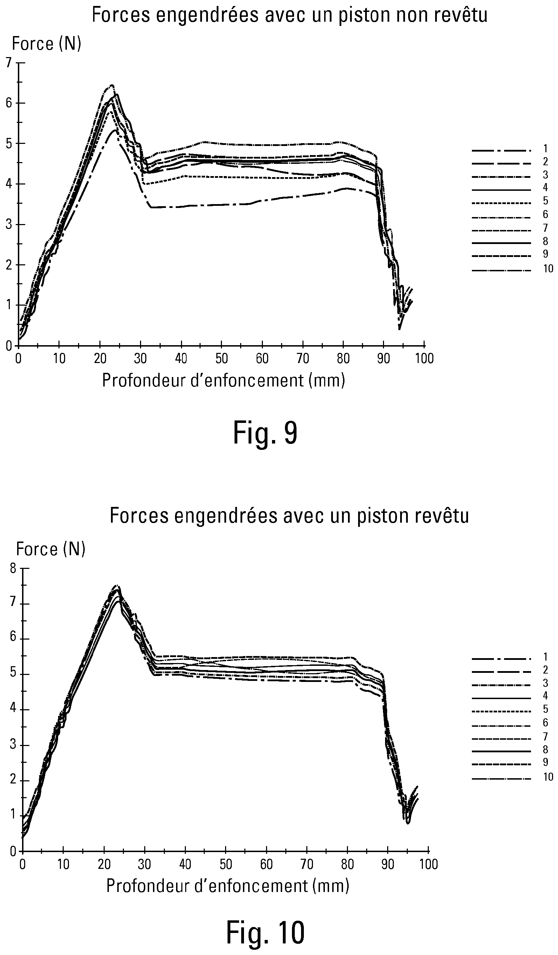

La présence du profil de déformation 133 dans la surface externe axiale frontale 131 de la paroi frontale 13 facilite cette insertion. En effet, la présence du revêtement 135 rigidifie le matériau et rend donc généralement son insertion plus compliquée. Les figures 8 et 9 démontrent que la présence d'un revêtement ETFE sur la surface externe axiale frontale 131 du piston augmente sensiblement la force nécessaire pour déplacer ledit piston dans le tube d'insertion 2. Ainsi, on constate une augmentation des forces d'activation et de glissement de l'ordre de 30-40% en défaveur du piston avec film. On passe ainsi d'une force d'activation d'environ 6N pour un piston sans revêtement à environ 8N avec un piston avec revêtement, et les forces de glissement passent d'environ 4N à environ 5.5N. Le fait de prévoir un profil de déformation permet donc de compenser cette augmentation de frottement liée au revêtement ETFE, et ainsi permet d'assurer une insertion plus fiable et sûre du bouchon-piston revêtu tel que décrit ci-dessus. De même, la présence du profil de déformation 133 dans la surface externe axiale frontale 131 de la paroi frontale 13 du piston avec revêtement permet d'avoir un comportement identique à celui d'un piston sans revêtement, notamment en ce qui concerne les forces d'activation et de glissement du piston lors de l'actionnement.

Avantageusement, comme visible sur les figures 1 et 4 et 11 à 14, le profil d'étanchéité 130 de la paroi frontale 13 est formé entre ledit profil de déformation 133 de la surface externe axiale frontale 131 et un évidement périphérique radialement externe 137. Cet évidement périphérique radialement externe 137 est décalé axialement vers l'arrière par rapport à ladite surface externe axiale frontale 131 et on constate sur les figure 2 et 12 que pendant et après l'insertion du bouchon-piston à l'intérieur du corps de seringue 1, cet évidement périphérique radialement externe 137 est également déformé, notamment radialement. Cette mise en œuvre favorise encore d'avantage les processus d'insertion et d'actionnement tout en assurant une parfaite étanchéité après insertion. En effet, ledit profil d'étanchéité 130 de la paroi frontale est sollicité radialement vers l'extérieur, et donc contre le corps de seringue 1, à la fois par le profil de déformation 133 élastiquement déformé et par l'évidement périphérique radialement externe 137 également élastiquement déformé.

Le volume interne 15 du bouchon-piston 10 peut comporter un filetage 115. Avantageusement, ce filetage 115 est discontinu. Cette mise en œuvre facilite le processus de moulage du bouchon-piston comme cela sera décrit ci-après. Avantageusement, plusieurs rainures axiales 116, avantageusement quatre, sont prévues pour interrompre ledit filetage 115. En se référant à la figure 6, qui illustre le noyau du moule permettant de définir le corps cylindrique creux 11 du bouchon-piston 10 de la figure 1, on constate que l'embout 211 du noyau 200 est pourvu d'un filet externe 215 qui est interrompu ou séparé par quatre rainures axiales 216. Ainsi, en comparaison au noyau 200 de la figure 5, dans lequel le filet 215 est continu, le noyau de la figure 6 permet de réaliser le filetage interrompu de la figure 1, ce qui facilite notamment le démoulage du bouchon-piston en diminuant les contre-dépouilles.

Les modes de réalisation des figure 4 et 13, avec le bouchon-piston 10 ayant une surface frontale plane et perpendiculaire à l'axe central, peut bien entendu également être réalisé avec ce filetage discontinu, si nécessaire.

La figure 14 illustre un bouchon 10 ayant une surface frontale plane et perpendiculaire à l'axe central, et dépourvu de filetage.

La figure 7 illustre une machine de fabrication du bouchon-piston de la figure 1, avec une première partie de moule 201 qui est la partie inférieure sur la figure 7 et une seconde partie du moule 202 qui est la partie supérieure. La première partie du moule 201 est utilisée pour former le corps cylindrique creux 11 et comporte à cet effet un noyau 200 tel que montré sur la figure 6. Ce noyau 200 comporte un corps 210 qui se prolonge par un embout axial 211 qui va définir ledit volume interne 15, et qui comporte donc ledit filet externe discontinu 215 décrit précédemment séparé par les quatre nervures axiales 216. La figure 8 illustre une vue en perspective de dessus de ladite première partie de moule 201 qui montre le noyau 200 à l'intérieur de la première partie de moule 201.

Bien entendu, un nombre quelconque de nervures axiales 216 peut être prévu. Si souhaité, le noyau de la figure 5 peut être utilisé avec un bouchon-piston conique selon la figure 1, ce qui permet de réaliser un filetage continu dans le volume interne 15.

De manière connue, le piston selon l'invention peut en outre être revêtu en totalité d'huile de silicone pour favoriser son glissement, notamment lors de son insertion dans la seringue. En variante, si l'on souhaite supprimer cette huile de silicone, on peut aussi prévoir un revêtement parylène, également sur la totalité du piston. Dans ce cas, le film ETFE sur la surface frontale du piston pourra être traité afin de permettre l'accroche du parylène.

Bien que la présente invention ait été décrite en référence à différents modes de réalisation, il est entendu que la présente invention n'est pas limitée par ceux-ci mais qu'au contraire l'homme du métier peut y apporter toutes modifications utiles sans sortir du cadre de la présente invention tel que défini par les revendications annexées.

## Revendications

1. Bouchon-piston (10), notamment en élastomère, comportant un corps cylindrique (11) ayant un côté axial avant fermé par une paroi frontale (13), ledit corps cylindrique (11) comportant une surface externe pourvue d'au moins un profil d'étanchéité (110), ladite paroi frontale (13) comportant une surface externe axiale frontale (131) pourvue d'un revêtement (135) sous forme de film en éthylène tétrafluoroéthylène, ladite paroi frontale (13) comportant sur son bord radialement externe un profil d'étanchéité (130), **caractérisé en ce que** ladite surface externe axiale frontale (131) de ladite paroi frontale (13) comporte un profil de déformation (133) disposé radialement à l'intérieur dudit profil d'étanchéité (130) de ladite paroi frontale (13), ledit profil de déformation (133) étant adapté à se déformer radialement pour faciliter l'insertion et/ou le glissement dudit bouchon-piston (10) dans un corps de seringue (1), ledit profil de déformation (133) étant de forme arrondie, de sorte que ladite surface externe axiale frontale (131) de ladite paroi frontale (13) est dépourvue d'angles vifs.

2. Bouchon-piston selon la revendication 1, dans lequel ledit corps cylindrique creux (11) comporte une pluralité de profils d'étanchéité (110), avantageusement deux.

3. Bouchon-piston selon la revendication 2, dans lequel ledit au moins un profil d'étanchéité (110) dudit corps cylindrique creux (11) est un bourrelet radialement saillant.

4. Bouchon-piston selon l'une quelconque des revendications précédentes, dans lequel ledit profil de déformation (133) est réalisé sous la forme d'une rainure périphérique ménagée dans ladite surface externe axiale frontale (131) de ladite paroi frontale (13), ladite rainure étant avantageusement en forme de V ou de U.

5. Bouchon-piston selon l'une quelconque des revendications précédentes, dans lequel ledit profil d'étanchéité (130) de ladite paroi frontale (13) est formé entre ledit profil de déformation (133) et un évidement périphérique radialement externe (137) axialement décalé vers l'arrière par rapport à ladite surface externe axiale frontale (131), ledit évidement périphérique radialement externe (137) étant également déformé lors de l'insertion et/ou l'actionnement dudit bouchon-piston (10) dans un corps de seringue (1).

6. Bouchon-piston selon l'une quelconque des revendications précédentes, dans lequel ledit corps cylindrique (11) est creux et définit un volume interne (15), ledit corps cylindrique creux (11) ayant un côté axial arrière ouvert (12) axialement opposé audit côté axial avant fermé par ladite paroi frontale (13).

7. Bouchon-piston selon la revendication 6, dans lequel ledit volume interne (15) coopère avec un organe d'actionnement d'un dispositif de seringue.

8. Bouchon-piston selon la revendication 7, dans lequel ledit corps cylindrique creux (11) comporte sur sa surface interne un filetage (115) adapté à se visser sur un filet dudit organe d'actionnement.

9. Bouchon-piston selon la revendication 8, dans lequel ledit filetage (115) est discontinu, une pluralité de rainures axiales (116), avantageusement quatre, réalisées dans la surface interne dudit corps cylindrique creux (11), étant prévues pour interrompre ledit filetage (115).

10. Bouchon-piston selon l'une quelconque des revendications précédentes, dans lequel ladite surface externe axiale frontale (131) est conique et définit une pointe axiale centrale (1310).

11. Bouchon-piston selon l'une quelconque des revendications 1 à 9, dans lequel ladite surface externe axiale frontale (131) est sensiblement plane et perpendiculaire à un axe central dudit piston-bouchon (10).

12. Dispositif de seringue, comportant un corps de seringue cylindrique (1) contenant un produit fluide, **caractérisé en ce que** ledit corps de seringue (1) reçoit de manière coulissante un bouchon-piston (10) selon l'une quelconque des revendications précédentes.

13. Dispositif selon la revendication 12, dans lequel ledit au moins un profil d'étanchéité (110) dudit corps cylindrique creux (11) et ledit profil d'étanchéité (130) de ladite paroi frontale (13) coopèrent de manière étanche avec la surface cylindrique interne dudit corps de seringue (1), ladite surface externe axiale frontale (131) étant en contact avec ledit produit fluide.

14. Dispositif selon la revendication 13, dans lequel ledit profil d'étanchéité (130) est au moins partiellement pourvu dudit revêtement (135), définissant ainsi une zone d'étanchéité cylindrique entre la surface cylindrique interne dudit corps de seringue (1) et ledit profil d'étanchéité (130) revêtu.

## Patentansprüche

1. Kolbenstopfen (10), insbesondere aus Elastomer, mit einem zylindrischen Körper (11), der auf einer axialen Vorderseite durch eine Stirnwand (13) verschlossen ist, wobei der zylindrische Körper (11) eine äußere Oberfläche mit mindestens einem Dichtprofil (110) aufweist, wobei die Stirnwand (13) eine axiale Stirnaußenfläche (131) mit einer Beschichtung (135) in Form eines Films aus Ethylen-Tetrafluorethylen aufweist, wobei die Stirnwand (13) an ihrem radial äußeren Rand ein Dichtprofil (130) aufweist,
**dadurch gekennzeichnet, dass**
die axiale Stirnaußenfläche (131) der Stirnwand (13) ein Verformungsprofil (133) aufweist, das radial innerhalb des Dichtprofils (130) der Stirnwand (13) angeordnet ist, wobei das Verformungsprofil (133) dazu eingerichtet ist, sich radial zu verformen, um das Einsetzen und/oder das Gleiten des Kolbenstopfens (10) in einen Spritzenkörper (1) zu erleichtern, wobei das Verformungsprofil (133) derart abgerundet ist, dass die axiale Stirnaußenfläche (131) der Stirnwand (13) frei von scharfen Kanten ist.

2. Kolbenstopfen nach Anspruch 1, wobei der hohle zylindrische Körper (11) eine Mehrzahl von Dichtprofilen (110) aufweist, vorzugsweise zwei.

3. Kolbenstopfen nach Anspruch 2, wobei das mindestens eine Dichtprofil (110) des hohlen zylindrischen Körpers (11) ein radial hervorstehender Wulst ist.

4. Kolbenstopfen nach einem der vorhergehenden Ansprüche, wobei das Verformungsprofil (133) als umlaufende Nut in der axialen Stirnaußenfläche (131) der Stirnwand (13) ausgebildet ist, wobei die Nut vorzugsweise V-förmig oder U-förmig ist.

5. Kolbenstopfen nach einem der vorhergehenden Ansprüche, wobei das Dichtprofil (130) der Stirnwand (13) zwischen dem Verformungsprofil (133) und einer radial äußeren umlaufenden Vertiefung (137) gebildet ist, die gegenüber der axialen Stirnaußenfläche (131) axial nach hinten versetzt ist, wobei die radial äußere umlaufende Vertiefung (137) ebenfalls beim Einsetzen und/oder Betätigen des Kolbenstopfens (10) in einem Spritzenkörper (1) verformt wird.

6. Kolbenstopfen nach einem der vorhergehenden Ansprüche, wobei der zylindrische Körper (11) hohl ist und ein Innenvolumen (15) definiert, wobei der hohle zylindrische Körper (11) eine offene axiale Rückseite (12) aufweist, die axial der durch die Stirnwand (13) verschlossenen axialen Vorderseite gegenüberliegt.

7. Kolbenstopfen nach Anspruch 6, wobei das Innenvolumen (15) mit einem Betätigungselement einer Spritzvorrichtung zusammenwirkt.

8. Kolbenstopfen nach Anspruch 7, wobei der hohle zylindrische Körper (11) auf seiner Innenfläche ein Gewinde (115) aufweist, das dazu eingerichtet ist, auf ein Gewinde des Betätigungselements aufgeschraubt zu werden.

9. Kolbenstopfen nach Anspruch 8, wobei das Gewinde (115) unterbrochen ist, wobei eine Mehrzahl axialer Nuten (116), vorzugsweise vier, in der Innenfläche des hohlen zylindrischen Körpers (11) ausgebildet ist, um das Gewinde (115) zu unterbrechen.

10. Kolbenstopfen nach einem der vorhergehenden Ansprüche, wobei die axiale Stirnaußenfläche (131) konisch ist und eine zentrale axiale Spitze (1310) definiert.

11. Kolbenstopfen nach einem der Ansprüche 1 bis 9, wobei die axiale Stirnaußenfläche (131) im Wesentlichen eben und senkrecht zu einer Mittelachse des Kolbenstopfens (10) ist.

12. Spritzvorrichtung mit einem zylindrischen Spritzenkörper (1), der ein flüssiges Produkt enthält, **dadurch gekennzeichnet, dass** der Spritzenkörper (1) einen Kolbenstopfen (10) nach einem der vorhergehenden Ansprüche gleitend aufweist.

13. Vorrichtung nach Anspruch 12, wobei das mindestens eine Dichtprofil (110) des hohlen zylindrischen Körpers (11) und das Dichtprofil (130) der Stirnwand (13) dicht mit der zylindrischen Innenfläche des Spritzenkörpers (1) zusammenwirken, wobei die axiale Stirnaußenfläche (131) mit dem flüssigen Produkt in Kontakt steht.

14. Vorrichtung nach Anspruch 13, wobei das Dichtprofil (130) zumindest teilweise mit der Beschichtung (135) versehen ist und dadurch eine zylindrische Dichtzone zwischen der zylindrischen Innenfläche des Spritzenkörpers (1) und dem beschichteten Dichtprofil (130) definiert.

## Claims

1. A stopper/piston (10), in particular made of elastomer, comprising a cylindrical body (11) having a front axial end that is closed by a front wall (13), said cylindrical body (11) including an outside surface that is provided with at least one sealing profile (110), said front wall (13) including a front axial outside surface (131) that is provided with a coating (135) being a film made of ethylene tetrafluoroethylene (ETFE), said front wall (13) including a sealing profile (130) on its radially-outer edge, the stopper/piston being **characterized in that** said front axial outside surface (131) of said front wall (13) includes a deformation profile (133) that is arranged radially inside said sealing profile (130) of said front wall (13), said deformation profile (133) being adapted to deform radially so as to make it easier to insert and/or to slide said stopper/piston (10) into a syringe body (1), said deformation profile (133) being of rounded shape, such that said front axial outside surface (131) of said front wall (13) does not have any sharp angle.

2. A stopper/piston according to claim 1, wherein said hollow cylindrical body (11) includes a plurality of sealing profiles (110), advantageously two.

3. A stopper/piston according to claim 2, wherein said at least one sealing profile (110) of said hollow cylindrical body (11) is a radially-projecting bead.

4. A stopper/piston according to any preceding claim, wherein said deformation profile (133) is made in the shape of a peripheral groove that is formed in said front axial outside surface (131) of said front wall (13), said groove advantageously being V-shaped or U-shaped.

5. A stopper/piston according to any preceding claim, wherein said sealing profile (130) of said front wall (13) is formed between said deformation profile (133) and a radially-outer peripheral recess (137) that is axially offset towards the rear relative to said front axial outside surface (131), said radially-outer peripheral recess (137) also being deformed while said stopper/piston (10) is being inserted into a syringe body (1) and/or while it is being actuated.

6. A stopper/piston according to any preceding claim, wherein said cylindrical body (11) is hollow and defines an internal volume (15), said hollow cylindrical body (11) having an open rear axial end (12) that is axially remote from said front axial end that is closed by said front wall (13).

7. A stopper/piston according to claim 6, wherein said internal volume (15) co-operates with an actuator member of a syringe device.

8. A stopper/piston according to claim 7, wherein, on its inside surface, said hollow cylindrical body (11) includes a screw thread (115) that is adapted to be engaged on a thread of said actuator member.

9. A stopper/piston according to claim 8, wherein said thread (115) is discontinuous, a plurality of axial grooves (116), advantageously four grooves, made in the inside surface of said hollow cylindrical body (11), being provided so as to interrupt said thread (115).

10. A stopper/piston according to any preceding claim, wherein said front axial outside surface (131) is conical and defines a central axial tip (1310).

11. A stopper/piston according to any one of claims 1 to 9, wherein said front axial outside surface (131) is substantially plane and perpendicular to a central axis of said stopper/piston (10).

12. A syringe device including a cylindrical syringe body (1) containing a fluid, the device being **characterized in that** said syringe body (1) slidably receives a stopper/piston (10) according to any preceding claim.

13. A device according to claim 12, wherein said at least one sealing profile (110) of said hollow cylindrical body (11) and said sealing profile (130) of said front wall (13) co-operate in leaktight manner with the cylindrical inside surface of said syringe body (1), said front axial outside surface (131) being in contact with said fluid.

14. A device according to claim 13, wherein said sealing profile (130) is provided, at least in part, with said coating (135), thereby defining a cylindrical sealing zone between the cylindrical inside surface of said syringe body (1) and said coated sealing profile (130).
